Europäisches Patentamt

European Patent Office

Office européen des brevets

(19)

(11) Veröffentlichungsnummer : **0 069 291**
**B1**

(12) # EUROPÄISCHE PATENTSCHRIFT

(45) Veröffentlichungstag der Patentschrift :
.18.09.85

(51) Int. Cl.⁴ : **C 12 P 7/56// C12N1/20**

(21) Anmeldenummer : 82105598.5

(22) Anmeldetag : 25.06.82

(54) Verfahren zur Herstellung optisch reiner D- oder L-Milchsäure.

(30) Priorität : 02.07.81 DE 3126021

(43) Veröffentlichungstag der Anmeldung :
12.01.83 Patentblatt 83/02

(45) Bekanntmachung des Hinweises auf die Patenterteilung : 18.09.85 Patentblatt 85/38

(84) Benannte Vertragsstaaten :
BE CH DE FR GB IT LI NL

(56) Entgegenhaltungen :
CH-A- 62 577
FR-A- 899 094
FR-A- 1 291 519
FR-A- 2 273 064
GB-A- 1 030 740
CHEMICAL ABSTRACTS, Band 65, Nr. 3, 01. August
1966, Spalte 4616, Auszug c, Columbus, Ohio, USA
CHEMICAL ABSTRACTS, Band 29, Nr. 4, 20. Februar
1935, Spalte 1128, Auszug 5, Columbus, Ohio, USA H.
KATAGIRI et al.: "Lactic acid bacteria isolated from
the yeast mashes for sake manufacture I. The fermentation products from koji extract"
CHEMICAL ABSTRACTS, Band 82, Nr. 9, 03. März
1975, Seite 445, Spalte 2, Nr. 56031w, Columbus,
Ohio, USA F. SHUNICHI et al.: "Preparation of yeast
extract by cell wall lytic enzymes"
CHEMICAL ABSTRACTS, Band 63, Nr. 3, 2. August
1965, Spalte 3348, Auszug e, Columbus, Ohio, USA R.
IRIE et al.: "Nutritional requirements for Lactobacillus bulgaricus. II. Growth limiting factors in yeast
extract-glucose broth"
CHEMICAL ABSTRACTS, Band 23, Nr. 16, 20. August
1929, Seite 3948, Absatz 5, Columbus, Ohio, USA J.M.
NEILL et al. : "The use of culture media from
commercial dried yeast as a routine substitute for
meat infusion peptone media"

(73) Patentinhaber : BASF Aktiengesellschaft
Carl-Bosch-Strasse 38
D-6700 Ludwigshafen (DE)

(72) Erfinder : Cooper, Bryan, Dr.
Bruesseler Ring 47
D-6700 Ludwigshafen (DE)
Erfinder : Kuesters, Werner, Dr.
Osloer Weg 46
D-6700 Ludwigshafen (DE)
Erfinder : Martin, Christoph, Dr.
Kolpingstrasse 6
D-6800 Mannheim (DE)
Erfinder : Siegel, Hardo, Dr.
Hans-Purrmann-Allee 25
D-6720 Speyer (DE)

**Beschreibung**

Die Erfindung betrifft ein neues Verfahren zur Herstellung optisch reiner D- oder L-Milchsäure durch Fermentation.

Die Herstellung von Milchsäure durch Vergärung von Zuckern mit Bakterien der Gattung Lactobacillus ist seit langer Zeit bekannt. Diese technischen Gärprozesse führen nicht zu optisch reiner Milchsäure, sondern zu racemischen Gemischen. Racemische Milchsäure wird in beträchtlichen Mengen in der Lebensmittelindustrie verwendet.

Als Ausgangsmaterial für die Herstellung optisch reiner Wirkstoffe für den Pharma- und Pflanzenschutzsektor eignet sich racemische Milchsäure jedoch nicht. Gesuchte Vorprodukte sind hier optisch reine D- bzw. L-Milchsäure.

Es ist bereits versucht worden, optisch reine Milchsäure mit Hilfe von Lactobacillus-Arten herzustellen. Diese benötigen für ihr Wachstum eine Reihe von Substanzen, die sie nicht selbst herstellen können, wie beispielsweise Biotin, Thiamin, Nicotinsäure, Pyridoxamin, p-Aminobenzoesäure, Pantothensäure und Cyanocobalamin. Diese Verbindungen werden in Form komplexer Substrate dem Nährmedium zugesetzt. So benützt man beispielsweise zur Kultivierung von Lactobacillen im Labormaßstab das von de Man, Rogosa und Sharpe (J. Appl. Bacteriol. 23, 130 (1960) entwickelte Komplexmedium (« MRS-Medium »), das folgende Bestandteile enthält : Pepton, Fleischextrakt, Hefeextrakt, Tween 80®, Natriumacetat, Triammoniumcitrat, $MgSO_4$, $MnSO_4$ und $K_2HPO_4$.

Für eine industrielle Herstellung von Milchsäure eignet sich dieses Medium jedoch nicht, da die verwendeten komplexen Substrate zu teuer sind und nicht in dem benötigten Maßstab in immer gleicher Qualität zur Verfügung stehen. Technischen Nährlösungen werden daher komplexe Substrate wie Zuckerrübenmelasse oder Maisquellwasser zugesetzt (DE-OS 16 42 738). Diese stimulieren zwar das Wachstum der Bakterien, eine Herstellung optisch reiner Milchsäure unter Verwendung dieser Komponenten ist jedoch nicht möglich, da diese selbst racemische Milchsäure in beträchtlicher Menge enthalten. Optisch reine Milchsäure kann aus racemisch verunreinigter Milchsäure jedoch nur durch aufwendige und umständliche Ausfällung und Umkristallisation der Salze von D- bzw. L-Milchsäure hergestellt werden. Einsetzungen GB-A-10 30 740 beschreibt ein Verfahren zur Herstellung optisch reiner D(-)-Milchsäure in Gegenwert von Hefeextrakt und FR-A-12 91 519 beschreibt ein für das Wachstum milchsäure bildender Bakterien geeignetes Medium. Das Medium schließt nicht nur Bierhefe, sondern auch Milchpulver, das alle für das Wachstum von Lactobacillus notwendigen Bestandteile enthält, ein.

Es wurde nun gefunden, daß man die Herstellung von optisch reiner Milchsäure sehr vereinfachen kann.

Gegenstand der Erfindung ist ein Verfahren zur Herstellung optisch reiner D- oder L-Milchsäure durch Fermentation eines wäßrigen Nährmediums, welches Stickstoff, Vitamine, Aminosäuren, Zucker und Spurenelemente enthält, mit Hilfe eines Mikroorganismus bei pH 4 bis 6, dadurch gekennzeichnet, daß das Nährmediums als Quelle für Stickstoff, Vitamine, Aminosäuren und Spurenelemente Bierhefe enthält.

Bierhefe enthält alle für die Fermentation benötigten Vitamine, Eiweißstoffe und Spurenelemente in ausreichender Konzentration. Als Bierhefe kommen beispielsweise Saccharomyces cerevisiae und S. carlsbergensis in Betracht.

Die Bierhefe fällt als Nebenprodukt bei der Bierherstellung an. Die Konzentration der Bierhefe im Nährmedium liegt zwischen 1 und 50, vorzugsweise 5 bis 30 g TS/l (TS = Trockensubstanz). Die Bierhefe kann in Form einer frischen wäßrigen Suspension mit ca. 10 % TS, wie sie von Brauereien abgegeben wird, oder auch in Form einer getrockneten Produktes, wie es von Bierhefeverwertungsfirmen angeboten wird, zum Einsatz kommen. In diesen Formen kann sie direkt verwendet werden, vorteilhaft ist jedoch, sie in Wasser mehrere Stunden auf 90 bis 100 °C zu erhitzen. In einer weiteren Ausführungsform des erfindungsgemäßen Verfahrens wird die Bierhefe in Wasser nei 30 bis 60 °C in einer Konzentration von 1 bis 10 % ein bis zwei Stunden inkubiert, um eine Autolyse einzuleiten. Eine Zugabe von proteolytisch wirkenden Enzymen ist möglich, jedoch nicht notwendig. Ein mechanischer Aufschluß mit einem Zellaufschlußgerät (beispielsweise Dynomill®) ist ebenfalls vorteilhaft.

Das Nährmedium muß als Kohlenstoffquelle einen von den Mikroorganismen verwertbaren Zucker enthalten, der zu Milchsäure abgebaut wird. Als Beispiele hierfür sind Saccharose, Lactose und Glucose zu nennen.

Als Mikroorganismus kann bei dem erfindungsgemäßen Verfahren zur Herstellung optisch reiner D- oder L-Milchsäure jeder Mikroorganismus eingesetzt werden, der jeweils nur ein Enantiomeres der Milchsäure bildet. Solche Mikroorganismen sind in den Beispielen genannt. Sie können von Hinterlegungsstellen für Mikroorganismen bezogen werden.

Der pH-Wert soll während der Fermentation des Zuckers bei etwa 4 bis 6, vorzugsweise 4,5 bis 5,5 liegen. Am einfachsten läßt sich dieser pH-Wert durch Zugabe von Calciumcarbonat in reiner oder technischer Form, z. B. als Schlämmkreide, gemahlener Kalkstein oder gemahlener Marmor, einstellen. Der pH-Wert kann aber auch durch Zugabe von Alkali- oder Erdalkalihydroxiden oder Alkalicarbonaten eingestellt werden.

Man führt die Fermentation im allgemeinen in einem temperierbaren Rührbehälter bei der für den Mikroorganismus optimalen Temperatur im Bereich von etwa 40 bis 60 °C durch. In einer bevorzugten Ausführungsform des erfindungsgemäßen verfahrens wird ein temperierbarer Rührbehälter mit Wasser, Bierhefe und Schlämmkreide beschickt. Diese Mischung wird ca. 4 h unter $N_2$ gekocht. Nach Abkühlung auf die Gärtemperatur wird Glucose zugegeben und die Mischung mit einer aktiv gärenden Vorkultur des verwendeten Mikroorganismus beimpft (Impfmenge 1 bis 20 %). Die Gärung wird abgebrochen, wenn die eingesetzte Glucose verbraucht ist. Die D- oder L-Milchsäure kann dann aus der Fermentations-Maische nach einer der üblichen Methoden isoliert werden. Beispielsweise kann die Maische mit Schwefelsäure auf pH 2 angesäuert und dann filtriert werden. Das Filtrat enthält die D- oder L-Milchsäure in optisch reiner Form. Durch Einengen des Filtrates erhält man die Milchsäure in guter chemischer Reinheit.

Das neue Verfahren liefert D- bzw. L-Milchsäure in fast vollständiger optischer Reinheit und in sehr guten Ausbeuten. Darüber hinaus ist das Verfahren sehr einfach durchführbar.

Folgende Beispiele sollen die Erfindung erläutern :

## Beispiel 1

320 g Schlämmkreide, 80 g getrocknete Bierhefe und 2,4 l Trinkwasser werden in einem 5 l-Glasfermenter unter Rühren und $N_2$-Begasung 4 H lang gekocht. Nach dem Abkühlen auf 45 °C werden 4 g konzentrierte Phosphorsäure sowie 400 g Glucosemonohydrat, die in 1,6 l Wasser 15 min bei 121 °C sterilisiert wurden, zugegeben und die Mischung mit 40 ml einer maximal 24 h alten Vorkultur von Lactobacillus lactis ATCC 8000 in MRS-Medium beimpft. Der Ansatz wird bei 45 °C unter $N_2$-Überlagerung gerührt. In regelmäßigen Abständen werden Proben entnommen und deren Gehalt an Milchsäure auf enzymatischem Weg bestimmt. Das Ergebnis ist wie folgt :

| Fermentationszeit (h) | D-Milchsäure (g/l) | L-Milchsäure (g/l) |
|---|---|---|
| 15,5 | 11 | |
| 44 | 34 | |
| 64 | 52 | |
| 88 | 72 | |
| 96 | 78 | |
| 112 | 83 | 0,3 |

Die Gärung ist nach 112 h beendet (Glucose-Konzentration 0). Die Kulturbrühe wird mit 300 g konzentrierter Schwefelsäure angesäuert und über ein Filtertuch abgesaugt. Nach Einengen des Filtrates erhält man 308 g D-Milchsäure, welche eine optische Reinheit von 99,3 % besitzt.

## Beispiel 2

4 l eines wie in Beispiel 1 beschriebenen Bierhefemediums werden mit 40 ml einer maximal 24 halten Vorkultur von Lactobacillus lactis DSM 20073 in MRS-Medium (45 °C) beimpft. Der Ansatz wird bei 45 °C unter $N_2$-Überlkagerung gerührt. Die Bildung der Milchsäure verläuft folgendermaßen :

| Fermentationszeit (h) | D-Milchsäure (g/l) | L-Milchsäure (g/l) |
|---|---|---|
| 15 | 37,0 | |
| 24 | 46,0 | |
| 40 | 59,0 | |
| 48 | 68,2 | |
| 64 | 81,8 | |
| 70 | 82,4 | 0 |

Die Gärung ist nach 70 h beendet (Glucose-Konzentration 0). Die Kulturbrühe wird mit 300 g konzentrierter Schwefelsäure angesäuert und über ein Filtertuch abgesaugt und eingeengt. Die optische Reinheit der erhaltenen D-Milchsäure beträgt 100 %.

## Beispiel 3

1 l eines wie in Beispiel 1 beschriebenen Bierhefemediums, das jedoch nur 50 g/l Glucose enthielt, wird mit 10 ml einer 8 h alten Vorkultur von Lactobacillus casei IFO 3425 in MRS-Medium (40 °C) beimpft. Der Ansatz wird bei 40 °C unter $N_2$-Überlagerung gerührt. Die Bildung der L-Milchsäure ist in Tabelle 1 beschrieben. Die Gärung ist nach 38 h beendet. Die Kulturlösung wird mit 45 g konzentrierter Schwefelsäure angesäuert, filtriert und eingeengt. Die optische Reinheit der erhaltenen L-Milchsäure beträgt über 99 %.

## Beispiel 4

1 l eines wie in Beispiel 3 beschriebenen Bierhefemediums wird mit 10 ml einer 8 h alten Vorkultur von Lactobacillus casei ssp. rhamnosus DSM 20021 in MRS-Medium (40 °C) beimpft. Der Ansatz wird bei 40 °C unter $N_2$-Überlagerung gerührt. Die Bildung der L-Milchsäure ist in Tabelle 1 beschrieben. Die Gärung ist nach 38 h beendet. Die Kulturlösung wird anschließend mit 45 g konzentrierter Schwefelsäure angesäuert, filtriert und eingeengt. Die optische Reinheit der erhaltenen L-Milchsäure liegt bei über 99 %.

### Tabelle 1

### Milchsäuregärungen mit L. casei JFO 3425 und DSM 20021

| Fermentationszeit (h) | L-Milchsäure (g/l) | | D-Milchsäure (g/l) | |
|---|---|---|---|---|
| | JFO 3425 | DSM 20021 | JFO 3425 | DSM 20021 |
| 0 | 0 | 0 | 0 | 0 |
| 23 | 14,2 | 12,2 | 0 | 0 |
| 38 | 47,3 | 45,2 | 0,2 | 0,1 |

### Patentansprüche

1. Verfahren zur Herstellung optisch reiner D- oder L-Milchsäure durch Fermentation eines wäßrigen Nährmediums, welches Stickstoff, Vitamine, Aminosäuren, Zucker und Spurenelemente enthält, mit Hilfe eines Mikroorganismus der jeweils nur ein Enantiomeres der Milchsäure bildet, bei pH 4 bis 6, dadurch gekennzeichnet, daß das Nährmedium als Quelle für Stickstoff, Vitamine, Aminosäuren und Spurenelemente Bierhefe enthält.

2. Verfahren nach Anspruch 1, dadurch gekennzeichnet, daß die Bierhefe in Form einer frischen Hefesuspension eingesetzt wird.

3. Verfahren nach Anspruch 1, dadurch gekennzeichnet, daß die Bierhefe in getrockneter Form eingesetzt wird.

4. Verfahren nach Anspruch 1, dadurch gekennzeichnet, daß die Bierhefe durch Inkubation in Wasser vorbehandelt wird.

5. Verfahren nach Anspruch 1, dadurch gekennzeichnet, daß die Bierhefe durch einen mechanischen Aufschluß vorbehandelt wird.

### Claims

1. A process for the preparation of optically pure D- or L-lactic acid by fermentation of an aqueous nutrient medium, which contains nitrogen, vitamins, aminoacids, sugars and trace elements, by means of a microorganism which produces only one enantiomer of lactic acid, at a pH of 4 to 6, wherein the nutrient medium contains brewers' yeast as the source of nitrogen, vitamins, aminoacids and trace elements.

2. A process as claimed in claim 1, wherein the brewers' yeast is employed in the form of a fresh yeast suspension.

3. A process as claimed in claim 1, wherein dry brewers' yeast is employed.

4. A process as claimed in claim 1, wherein the brewers' yeast is pretreated by incubation in water.

5. A process as claimed in claim 1, wherein the brewers' yeast is pretreated by subjecting it to mechanical fragmentation.

**Revendications**

1. Procédé de préparation d'acide lactique -d ou -l optiquement pur, par fermentation d'un milieu nutritif aqueux, qui contient azote, vitamines, amino-acides, sucre et oligo-éléments, à l'aide d'un micro-organisme qui ne forme chaque fois qu'un énantiomère de l'acide lactique, à un pH de 4 à 6, caractérisé par le fait que le milieu nutritif ne contient, comme source d'azote, vitamines, amino-acides et oligo-éléments, que de la levure de bière.

2. Procédé selon la revendication 1, caractérisé par le fait que la levure de bière est introduite sous forme d'une suspension de levure fraîche.

3. Procédé selon la revendication 1, caractérisé par le fait que la levure de bière est introduite sous forme séchée.

4. Procédé selon la revendication 1, caractérisé par le fait que la levure de bière est prétraitée par incubation dans l'eau.

5. Procédé selon la revendication 1, caractérisé par le fait que la levure de bière est prétraitée par une désagrégation mécanique.